# FASCICULE DE BREVET EUROPEEN

(11) **EP 2 977 671 B1**
(45) Date de publication et mention de la délivrance du brevet: **04.09.2019**
(21) Numéro de dépôt: 14306197.6
(22) Date de dépôt: 24.07.2014
(51) Int. Cl.: F17C 13/00

(54) **Ensemble de distribution de gaz médical**
Anordnung zur Verteilung von medizinischem Gas
Medical gas distribution assembly

(43) Date de publication de la demande: 27.01.2016
(73) Titulaire: L'AIR LIQUIDE, SOCIETE ANONYME POUR L'ETUDE ET L'EXPLOITATION DES PROCEDES GEORGES CLAUDE, 75007 Paris (FR)
(72) Inventeur: Frenal, Antoine, 95460 Ezanville (FR); Gravière, Vincent, 92120 Montrouge (FR); Ligonesche, Renaud, 95220 Herblay (FR); Tarantello, Chiara, 92800 Puteaux (FR); Trevisan, Adrien, 92200 Neuilly sur Seine (FR)
(74) Mandataire: Pittis, Olivier

(56) Documents cités:
- US-A- 4 356 988
- US-A- 5 922 158

## Description

La présente invention concerne un ensemble de distribution de gaz comprenant un dispositif-support d'équipement apte à porter, de manière stable et en sécurité, un équipement médical, tel un humidificateur de gaz, sur une bouteille de gaz médical.

Un gaz respiratoire ou gaz médical qui sort d'une bouteille de gaz est généralement sec et a donc besoin d'être humidifié. Ceci est couramment opéré au moyen d'un humidificateur de gaz raccordé en sortie de la bouteille qui est traversé par le gaz, lequel se charge en vapeur d'eau avant d'être envoyé vers les voies aériennes du patient.

Or, actuellement, les bouteilles de gaz médicales ne présentent pas un système d'accroche d'équipement médical, en particulier d'humidificateur de gaz, qui soit sécurisé, stable et facile à mettre en oeuvre.

Ainsi, on connait des systèmes de fixation d'humidificateur ou analogue comprenant une ou plusieurs sangles venant ceinturer l'humidificateur sur le corps de la bouteille de gaz, c'est-à-dire sur son corps cylindrique. Ce type de système n'est pas très pratique à utiliser et surtout ne protège absolument pas l'humidificateur en cas de chute de la bouteille sur le sol.

On connait aussi des humidificateurs formés d'un bocal contenant de l'eau, venant se raccorder au raccord ou à la tuyauterie en sortie de bouteille, en y étant simplement suspendus. Là encore, un tel système ne protège absolument pas l'humidificateur en cas de chute de la bouteille sur le sol et on assiste alors, du fait d'une telle fixation instable, à son débranchement et/ou à sa rupture. On connait aussi de US5922159 une bouteille de gaz associée à un dispositif-support.

Le problème à résoudre est de proposer un ensemble de distribution de gaz amélioré comprenant un dispositif-support d'équipement permettant de recevoir et supporter un équipement médical, tel un humidificateur de gaz ou analogue, sur une bouteille de gaz et qui permette de protéger au moins partiellement ledit équipement, en cas de chute de l'ensemble sur le sol et qui puisse préférentiellement accueillir et supporter des équipements de tailles et formes différentes

La solution est un ensemble de distribution de gaz comprenant :
- une bouteille de gaz équipée d'un bloc de distribution de gaz,
- un capotage de protection agencé autour dudit bloc de distribution de gaz et
- un dispositif-support d'équipement apte à et conçu pour porter un équipement médical, le dispositif-support d'équipement comprenant :
   - une structure de fixation permettant d'accrocher le dispositif-support d'équipement au capotage de protection, la structure de fixation comprenant un premier bras de fixation et un second bras de fixation agencés face à face l'un de l'autre,
   - une structure-plateau sur laquelle peut être disposé ledit équipement à porter,
   - un ou plusieurs éléments de jonction reliant la structure de fixation à la structure-plateau, et
   - au moins une structure de maintien fixée à la structure-plateau et permettant un maintien en position dudit équipement sur ladite structure-plateau,
- et dans lequel le dispositif-support d'équipement est fixé audit capotage de protection par intermédiaire desdits premier et second bras de fixation, lesdits premier et second bras de fixation comprenant des portions d'extrémité venant enserrer le capotage à la manière d'un collier de serrage.

Selon le cas, l'ensemble de distribution de gaz de l'invention peut comprendre l'une ou plusieurs des caractéristiques techniques suivantes :
- les deux bras de fixation sont munis à leurs extrémités en contact avec le capotage, de prolongateurs de serrage conçus pour exercer une force de serrage sur le capotage.
- les portions d'extrémité des premier et second bras de fixation ou les prolongateurs de serrage équipant lesdits premier et second bras de fixation sont conformés pour épouser le contour extérieur du capotage.
- les portions d'extrémité des premier et second bras de fixation ou les prolongateurs viennent se loger dans des logements aménagés dans le capotage de protection.
- les prolongateurs sont des éléments additionnels fixés aux portions d'extrémité des premier et second bras de fixation.
- les prolongateurs sont des éléments additionnels en matériau polymère, par exemple en plastique.
- les prolongateurs sont des éléments additionnels formant des manchons autour des portions d'extrémité des premier et second bras de fixation.
- il comprend un premier et un second élément de jonction reliant la structure de fixation à la structure-plateau.
- la structure-plateau comprend un premier et un second bras-porteur.
- le premier élément de jonction est fixé au ou formé d'une seule pièce avec le premier bras de fixation de la structure de fixation et/ou avec le premier bras-porteur de la structure-plateau.
- le second élément de jonction est fixé au ou formé d'une seule pièce avec le second bras de fixation de la structure de fixation et/ou avec le second bras-porteur de la structure-plateau.
- lesdits premier et second bras-porteurs sont reliés l'un à l'autre par tout ou partie de la structure de maintien.
- les prolongateurs sont conçus pour exercer une force de serrage sur le capotage au niveau du logement, c'est-à-dire qu'ils prennent le capotage en sandwich.
- le bloc de distribution de gaz est un robinet à détendeur de gaz intégré ou RDI.
- le capotage de protection est rigide, de préférence en matériau polymère, tel un plastique, ou en métal.
- le capotage de protection comprend une poignée de portage, de préférence une poignée de portage surmontant le corps du capotage.
- le capotage de protection comprend aussi, de préférence sur sa face arrière, une poignée d'accrochage, avantageusement pivotante, permettant d'accrocher ou d'arrimer l'ensemble à un support, tel un barreau de lit ou un brancard hospitalier.
- la structure de maintien permet d'assurer un maintien en position de l'équipement sur la structure-plateau, lorsque ledit équipement est positionné sur la structure-plateau, en venant exercer une force de poussée, c'est-à-dire appuyer, sur ledit équipement en direction des éléments de jonction de manière à le prendre en sandwich et le maintenir ainsi en position fixe.
- la structure de maintien a une forme d'arceau.
- un ou plusieurs dispositifs de retenue reliant la structure de maintien aux éléments de jonction longilignes de manière à sécuriser l'équipement sur la structure-plateau.
- le ou les dispositifs de retenue sont choisis parmi les bandes, les liens, les cordons, les chainettes ou similaires.

Un ensemble de distribution de gaz selon l'invention est utilisable en milieu hospitalier, à domicile ou encore dans des véhicules d'urgence de type SAMU, pompiers, ambulances... pour distribuer du gaz médical, tel de l'oxygène, de l'air, de l'air enrichi en oxygène, des mélanges N₂O/O₂ ou tout autre gaz, à un patient, en particulier du gaz humidifié.

L'invention concerne donc alors aussi une utilisation d'un ensemble de distribution de gaz selon l'invention pour porter un équipement médical, ledit équipement médical étant disposé sur la structure-plateau en y étant maintenu par la structure de maintien.

De préférence, l'équipement médical est un humidificateur de gaz, en particulier un humidificateur de gaz raccordé fluidiquement à un raccord de sortie de gaz du bloc de distribution de gaz agencé sur la bouteille de gaz.

En outre, l'invention concerne également une installation de fourniture de gaz respiratoire à un patient comprenant :
- un ensemble de distribution de gaz selon l'invention comprenant une bouteille de gaz équipée d'un bloc de distribution de gaz, un capotage de protection agencé autour dudit bloc de distribution de gaz, et un dispositif-support d'équipement sur lequel est disposé un humidificateur de gaz relié fluidiquement à un raccord e sortie du bloc de distribution de gaz, et
- une canalisation souple reliée fluidiquement à une sortie de l'humidificateur,
- et une interface de distribution de gaz au patient reliée fluidiquement à la canalisation souple.

De préférence, l'interface de distribution de gaz comprend un masque respiratoire, nasal, buccal, facial ou narinaire, ou des lunettes respiratoires.

La présente invention va maintenant être décrite plus en détail en références aux Figures annexées parmi lesquelles :
- les Figures 1 à 4 représentent un premier mode de réalisation d'un ensemble de distribution de gaz médical selon l'invention,
- les Figures 5 à 7 représentent un deuxième mode de réalisation d'un ensemble de distribution de gaz médical selon l'invention,
- la Figure 8 schématise un premier mode de réalisation du dispositif-support d'équipement d'un ensemble selon l'invention, et
- les Figures 9A à 9C schématisent des modes de réalisation alternatifs du dispositif-support d'équipement d'un ensemble selon l'invention.

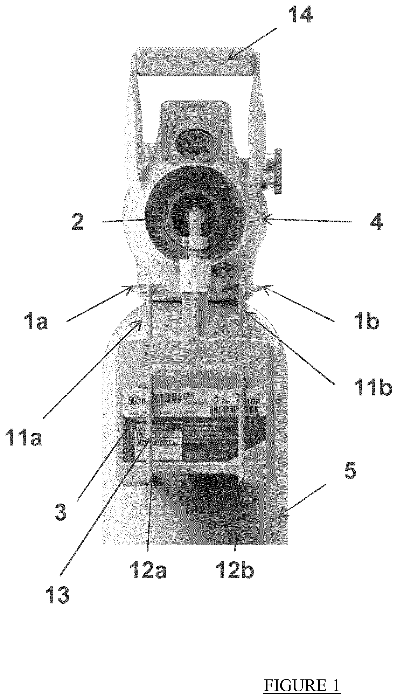

Plus précisément, les Figures 1 à 4 représentent un premier mode de réalisation d'un ensemble de distribution 1, 2, 4, 5 de gaz médical selon l'invention.

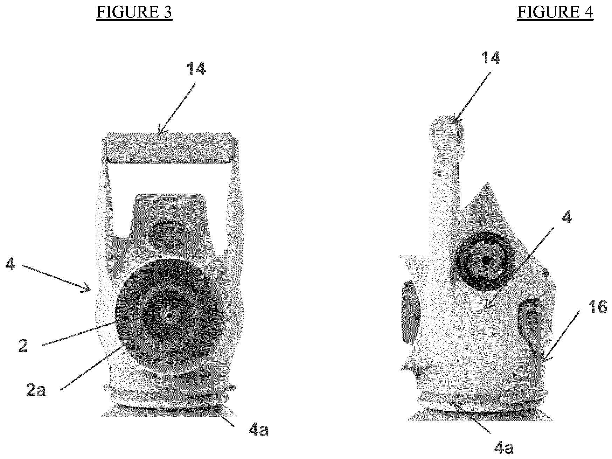

L'ensemble de distribution comprend une bouteille 5 de gaz médical, par exemple une bouteille de forme cylindrique contenant de l'oxygène ou un mélange N₂O/O₂, équipée d'un bloc de distribution 2 de gaz, tel un robinet ou robinet détendeur de gaz, de préférence un robinet à détendeur de gaz intégré ou RDI. Il comprend les différents organes et systèmes de réglage (manomètre, volant rotatif de réglage de débit...) permettant de contrôler la sortie du gaz et préférentiellement sa pression. La sortie de gaz se fait via un raccord de sortie 2a du bloc 2 qui est accessible via la face avant du capotage 4, comme montré en Figure 3.

Le bloc de distribution 2 de gaz est fixé de façon classique au niveau du col de la bouteille et est protégé par un capotage de protection 4, par exemple en polymère ou en métal, lequel est agencé autour dudit bloc de distribution 2 de gaz.

Ce capotage 4 forme une coque qui est en générale rigide. Il comprend une poignée de portage 14 pour faciliter le transport et les manipulations de la bouteille 5 de gaz. Des évidements pratiqués dans le corps du capotage 4 donnent accès aux différents organes et systèmes de réglage du bloc formant robinet ou robinet détendeur de gaz.

Le capotage 4 comporte aussi, sur sa face arrière, une poignée d'accrochage 16 pivotante permettant d'accrocher ou d'arrimer l'ensemble bouteille/capotage à un support, tel un barreau de lit, à un brancard, à une tringle-support d'un véhicule d'urgences ou analogue. Cette poignée d'accrochage 16 est schématisée en position repliée sur la Figure 4 qui montre le capotage 4 en vue latérale.

Par ailleurs, l'ensemble de distribution 1, 2, 4, 5 de gaz médical selon l'invention comprend aussi un dispositif-support d'équipement 1, 11, 12, 13 apte à et conçu pour porter un équipement médical 3, en particulier un humidificateur de gaz venant se raccorder au niveau du raccord de sortie 2a du bloc de distribution 2 de gaz de manière à permettre d'opérer une humidification du gaz sortant du corps cylindrique de la bouteille 5 avant son administration par inhalation à un patient.

Comme on le voit, le dispositif-support d'équipement 1, 11, 12, 13 comprend :
- une structure de fixation 1, 1a, 1b permettant d'accrocher le dispositif-support d'équipement 1, 11, 12, 13 au capotage de protection 4,
- une structure-plateau 12, 12a, 12b sur laquelle peut être disposé ledit équipement 3 à porter, de préférence elle comprend au moins un premier et un second bras-porteur 12a, 12b, par exemple agencé face à face ou en parallèle l'un de l'autre,
- un ou plusieurs éléments de jonction 11, 11a, 11b reliant la structure de fixation 1, 1a, 1b à la structure-plateau 12, 12a, 12b, par exemple elle comprend au moins deux éléments de jonction longilignes, telles des tiges, agencés en parallèle,
- au moins une structure de maintien 13, tel un arceau, fixée à la structure-plateau 12, 12a, 12b et permettant d'assurer un maintien en position dudit équipement 3 sur ladite structure-plateau 12, 12a, 12b, c'est-à-dire que l'équipement 3, lorsqu'il est positionné sur la structure-plateau 12, 12a, 12b, est pris en sandwich entre la structure de maintien 13 et le ou les éléments de jonction 11, 11a, 11b.

Avantageusement, l'ensemble formant le dispositif-support 1, 11, 12, 13 comprenant la structure de fixation 1, 1a, 1b, la structure-plateau 12, 12a, 12b, les éléments de jonction 11, 11a, 11b, et la structure de maintien 13 sont formés à partir d'une ou plusieurs pièces, avantageusement à partir d'une seule et même pièce.

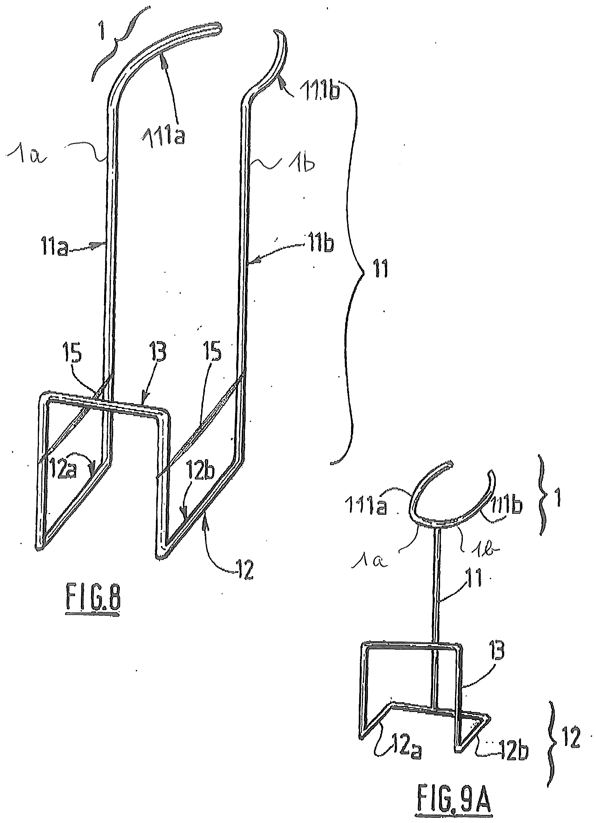

Par exemple, le dispositif-support 1, 11, 12, 13 est obtenu à partir d'une tige métallique conformée et/ou pliée sous forme désirée, ou d'une pièce en matière plastique, notamment en plastique moulé, tel qu'illustré en Figure 8. Il peut aussi être formé de plusieurs pièces ou sous-unités assemblées les unes aux autres, par soudage, collage ou tout autre moyen.

La matière plastique utilisable pour réaliser tout ou partie du dispositif-support 1, 11, 12, 13 est préférentiellement choisie parmi le PVC, le PE, le PET, le PP, le PMMA, le PU...

De façon alternative, lorsque le dispositif-support 1, 11, 12, 13 est formé d'un fil métallique (cf. Figure 8), par exemple un fil en acier, celui-ci peut être enrobé de Rilsan ou d'une autre matière analogue.

Comme on le comprend, dans le premier mode de réalisation des Figures 1 à 4, la structure de fixation 1, 1a, 1b comprend deux bras de fixation, à savoir un premier la et second bras 1b de fixation, venant se loger dans un ou des logements 4a aménagés dans le capotage 4 de protection. Le logement 4a est par exemple une gorge aménagée sur tout ou partie de la périphérie du capotage 4, comme détaillé en Figure 3.

Plus précisément, lorsque les deux bras de fixation 1a, 1b sont insérés dans le logement ou gorge 4a du capotage 4, ceux-ci prennent le capotage 4 en sandwich en l'enserrant, par exemple par clipsage. Le logement ou gorge 4a est donc conformé et dimensionné pour recevoir les deux bras de fixation 1a, 1b et assurer leur maintien en position dans ledit logement ou gorge 4a.

En d'autres termes, les deux bras de fixation 1a, 1b comprennent des portions d'extrémité 111a, 111b venant enserrer le capotage 4 à la manière d'un collier de serrage, c'est-à-dire en exerçant sur le capotage des forces de serrage permettant le maintien en position du dispositif-support d'équipement 1, 11, 12, 13.

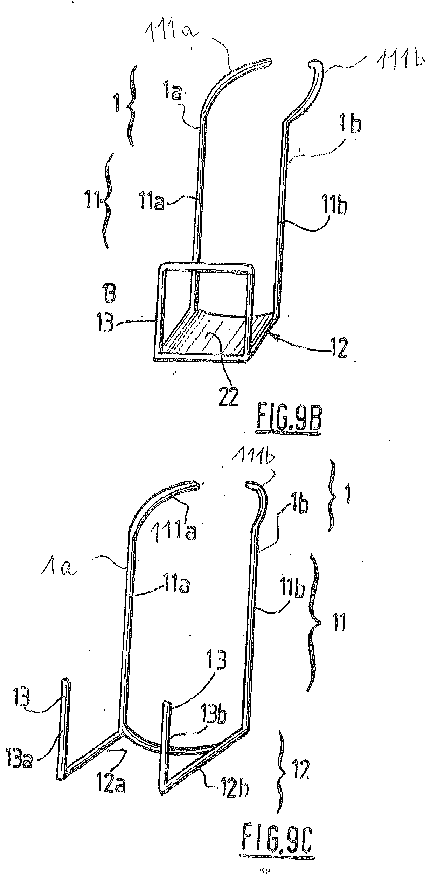

Les portions d'extrémité 111a, 111b sont les régions d'extrémité libre de chacun des deux bras de fixation 1a, 1b, comme illustré sur les Figures 9B et 9C notamment. Typiquement, les portions d'extrémité 111a, 111b ont une longueur comprise entre 4 et 30 cm environ.

Selon une variante de réalisation, les deux bras de fixation 1a, 1b peuvent aussi comprendre, à l'extrémité des portions d'extrémité 111a, 111b, des portions terminales ou extrémités recourbées (non montrés) de manière à faire saillie vers le bloc 2, lesquelles portions terminales viendraient se loger dans les logements ou orifices (non montrés) adaptés pour renforcer la fixation et la solidarisation du dispositif-support 1, 11, 12, 13 au capotage 4.

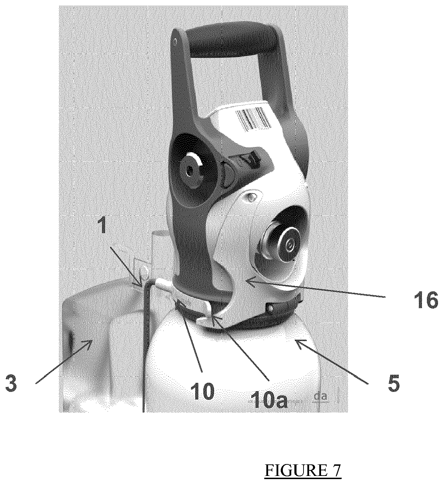

De façon générale, les deux bras de fixation 1a, 1b sont avantageusement conformés, au niveau des portions d'extrémité 111a, 111b, pour épouser au mieux le contour extérieur du capotage 4, en particulier au niveau du logement ou gorge périphérique 4a. Ainsi, les portions d'extrémité 111a, 111b des deux bras de fixation 1a, 1b sont préférentiellement légèrement arqués ou courbes pour épouser les contours courbes ou arrondis des capotages 4 des Figures 1 à 7. Toutefois, d'autres formes sont possibles, c'est-à-dire que les deux bras de fixation 1a, 1b peuvent aussi être conformés pour ne pas ou pas exactement ou totalement épouser le contour du capotage 4.

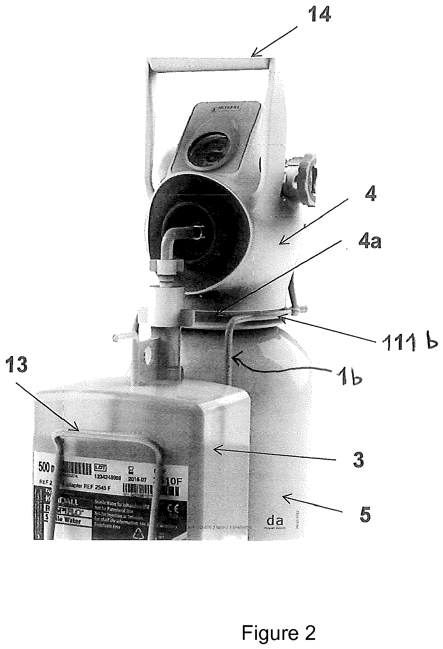

L'équipement 3, en particulier un humidificateur de gaz, peut alors être positionné sur la structure-plateau 12, 12a, 12b, en étant alors pris en sandwich entre la structure de maintien 13 et le ou les éléments de jonction 11, 11a, 11b, comme visible sur les Figures 1 et 2, puis raccordé au raccord de sortie 2a du bloc 2.

Optionnellement, afin d'éviter un glissement latéral de l'équipement 3 lorsqu'il est positionné sur la structure-plateau 12, 12a, 12b, on peut prévoir un ou plusieurs dispositifs de retenue 15, tel que bandes, liens, cordons... en matériau élastique, en tissu ou autre, permettant de sécuriser l'équipement 3, par exemple des dispositifs de retenue reliant la structure de maintien 13, tel un arceau, aux éléments de jonction 11, 11a longilignes, comme schématisé en Figure 8.

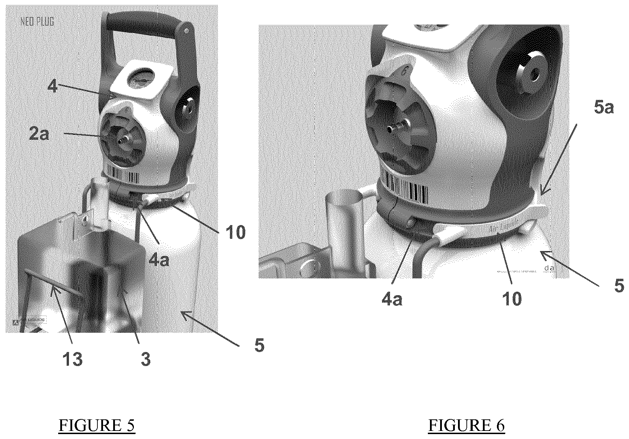

Les Figures 5 et 6 représentent un second mode de réalisation d'un ensemble de distribution de gaz médical selon l'invention, dans lequel la structure de fixation 1, 1a, 1b a été dotée d'un système adaptateur 10, 10a de façon à pouvoir s'adapter à différents modèles de capotage 4 de protection, c'est-à-dire à des formes de capotage 4 différentes.

Plus précisément, les deux bras de fixation 1a, 1b sont munis à leurs extrémités en contact avec le capotage 4, c'est-à-dire au niveau des portions d'extrémité 111a, 111b, de prolongateurs de serrage 10, encore appelés adaptateurs, venant enserrer le capotage 4, de part et d'autre, à la manière d'un de collier de serrage.

De préférence, les prolongateurs 10 exercent une force de serrage sur le capotage 4 au niveau du logement ou gorge périphérique 4a.

Afin de faciliter la mise en place du dispositif-support d'équipement 1, 11, 12, 13 lorsqu'il est équipé de tels prolongateurs de serrage 10, il est prévu que les extrémités 10a de ces prolongateurs 10 soient légèrement recourbées vers l'extérieur de manière à faciliter leur écartement par l'utilisateur et la mise en position subséquente du dispositif-support ou, à l'inverse, sa dépose. Les prolongateurs de serrage 10 sont préférentiellement réalisés en matière plastique.

Il est à noter que des modes de réalisations alternatifs possibles du dispositif-support 1, 11, 12, 13 sont illustrés en Figure 9A à 9C.

Ainsi, sur la Figure 9A, la structure de fixation 1 permettant d'accrocher le dispositif-support d'équipement 1, 11, 12, 13 au capotage de protection 4 comprend un bras unique 11.

Sur la Figure 9B, la structure-plateau 12 sur laquelle est disposé l'équipement 3 comprend un fond plein 22.

Sur la Figure 9C, la structure de maintien 13 fixée à la structure-plateau 12 qui permet de maintenir l'équipement en position sur la structure-plateau 12, 12a, 12b, comprend non pas un arceau comme illustré sur les Figures 8, 9A et 9B notamment, mais deux expansions 13a, 13b se projetant vers le haut. Il est à noter que d'autres formes d'expansions sont possibles et que leur nombre peut être plus élevé.

Il est à noter que des combinaisons d'éléments et/ou de formes issus des Figures 8, 9A à 9C sont également possibles pour réaliser d'autres variantes du dispositif-support.

Dans le premier mode de réalisation, ce sont les portions d'extrémité 111a, 111b des deux bras de fixation 1a, 1b qui exercent une action ou force de serrage directement sur le capotage 4 (cf. Fig. 1 et 2), alors que dans le second mode de réalisation, ce sont les prolongateurs de serrage 10 qui équipent les portions d'extrémité 111a, 111b des deux bras de fixation 1a, 1b qui exercent ladite action ou force de serrage sur le capotage 4 (cf. Fig. 5 et 6).

Le dispositif-support d'équipement de l'invention permet de fixer facilement, de manière stable et en sécurité un humidificateur de gaz ou un autre équipement médical, à une bouteille de gaz médical, de manière à minimiser toute situation d'instabilité et d'éviter ou limiter une détérioration de l'équipement en cas de chute accidentelle sur le sol de l'ensemble de distribution de gaz.

## Revendications

1. Ensemble de distribution de gaz (1, 2, 4, 5) comprenant :
- une bouteille (5) de gaz équipée d'un bloc de distribution (2) de gaz,
- un capotage de protection (4) agencé autour dudit bloc de distribution (2) de gaz,
- un dispositif-support d'équipement (1, 11, 12, 13) apte à et conçu pour porter un équipement médical (3), le dispositif-support d'équipement (1, 11, 12, 13) comprend :
• une structure de fixation (1, 1a, 1b) permettant d'accrocher le dispositif-support d'équipement (1, 11, 12, 13) au capotage de protection (4), la structure de fixation (1, 1a, 1b) comprenant un premier bras de fixation (1a) et un second bras de fixation (1b) agencés face à face l'un de l'autre,
• une structure-plateau (12, 12a, 12b) sur laquelle peut être disposé ledit équipement (3) à porter,
• un ou plusieurs éléments de jonction (11, 11a, 11b) reliant la structure de fixation (1, 1a, 1b) à la structure-plateau (12, 12a, 12b), et
• au moins une structure de maintien (13) fixée à la structure-plateau (12, 12a, 12b) et permettant un maintien en position dudit équipement (3) sur ladite structure-plateau (12, 12a, 12b),
- et dans lequel le dispositif-support d'équipement (1, 11, 12, 13) est fixé audit capotage de protection (4) par intermédiaire desdits premier (1a) et second bras (1b) de fixation, **caractérisé en ce que** lesdits premier (1a) et second bras (1b) de fixation comprennent des portions d'extrémité (111a, 111b) venant enserrer le capotage (4) à la manière d'un collier de serrage.

2. Ensemble selon la revendication précédente, **caractérisé en ce que** les deux bras de fixation (1a, 1b) sont munis à leurs extrémités (111a, 111b) en contact avec le capotage (4) de prolongateurs de serrage (10) conçus pour exercer une force de serrage sur le capotage (4).

3. Ensemble selon l'une des revendications précédentes, **caractérisé en ce que** les portions d'extrémité (111a, 111b) des premier (1a) et second bras (1b) de fixation ou les prolongateurs de serrage (10) équipant lesdits premier (1a) et second bras (1b) de fixation sont conformés pour épouser le contour extérieur du capotage (4).

4. Ensemble selon l'une des revendications précédentes, **caractérisé en ce que** les portions d'extrémité (111a, 111b) des premier (1a) et second bras (1b) de fixation ou les prolongateurs (10) viennent se loger dans des logements (4a) aménagés dans le capotage de protection (4).

5. Ensemble selon l'une des revendications précédentes, **caractérisé en ce qu'**il comprend un premier et un second élément de jonction (11a, 11b) reliant la structure de fixation (1, 1a, 1b) à la structure-plateau (12, 12a, 12b).

6. Ensemble selon l'une des revendications précédentes, **caractérisé en ce que** la structure-plateau (12, 12a, 12b) comprend un premier et un second bras-porteur (12a, 12b).

7. Ensemble selon l'une des revendications précédentes, **caractérisé en ce que** le premier élément de jonction (11a) est fixé au ou formé d'une seule pièce avec le premier bras de fixation (1a) de la structure de fixation (1, 1a, 1b) et/ou avec le premier bras-porteur (12a) de la structure-plateau (12, 12a, 12b), et/ou le second élément de jonction (11b) est fixé au ou formé d'une seule pièce avec le second bras de fixation (1b) de la structure de fixation (1, 1a, 1b) et/ou avec le second bras-porteur (12b) de la structure-plateau (12, 12a, 12b).

8. Ensemble selon l'une des revendications précédentes, **caractérisé en ce que** lesdits premier et second bras-porteurs (12a, 12b) sont reliés l'un à l'autre par tout ou partie de la structure de maintien (13).

9. Ensemble selon l'une des revendications précédentes, **caractérisé en ce qu'**il comprend un équipement médical (3) disposé sur la structure-plateau (12, 12a, 12b) en y étant maintenu par la structure de maintien (13).

10. Ensemble selon la revendication 9, **caractérisé en ce que** l'équipement médical (3) disposé sur la structure-plateau (12, 12a, 12b) est un humidificateur de gaz raccordé fluidiquement à un raccord de sortie de gaz du bloc de distribution (2) de gaz agencé sur la bouteille (5) de gaz.

11. Ensemble selon l'une des revendications précédentes, **caractérisé en ce qu'**un ou plusieurs dispositifs de retenue (15), choisis parmi les bandes, les liens, les cordons et les chainettes, relient la structure de maintien (13) aux premier et second éléments de jonction longilignes (11a, 11b).

12. Utilisation d'un ensemble selon l'une des revendications précédentes pour porter un équipement médical (3), ledit équipement médical (3) étant disposé sur la structure-plateau (12, 12a, 12b) en y étant maintenu par la structure de maintien (13).

13. Utilisation selon la revendication 12, **caractérisée en ce que** l'équipement médical (3) est un humidificateur de gaz.

14. Utilisation selon la revendication 13, **caractérisée en ce que** l'équipement médical (3) est un humidificateur de gaz raccordé fluidiquement à un raccord de sortie (2a) de gaz du bloc de distribution (2) de gaz agencé sur la bouteille (5) de gaz.

15. Installation de fourniture de gaz respiratoire à un patient comprenant :
- un ensemble de distribution de gaz (1, 2, 4, 5) selon l'une des revendications 1 à 11 comprenant une bouteille (5) de gaz équipée d'un bloc de distribution (2) de gaz, un capotage de protection (4) agencé autour dudit bloc de distribution (2) de gaz, et un dispositif-support d'équipement (1, 11, 12, 13) sur lequel est disposé un humidificateur de gaz (3) relié fluidiquement à un raccord de sortie (2a) du bloc de distribution (2) de gaz, et
- une canalisation souple reliée fluidiquement à une sortie de l'humidificateur (3),
- et une interface de distribution de gaz au patient reliée fluidiquement à la canalisation souple, de préférence l'interface de distribution de gaz comprend un masque respiratoire ou des lunettes respiratoires.

## Patentansprüche

1. Anordnung zur Ausgabe von Gas (1, 2, 4, 5), umfassend:
- eine Gasflasche (5), die mit einem Block zur Ausgabe (2) von Gas ausgerüstet ist,
- eine Schutzabdeckung (4), die um den Block zur Ausgabe (2) von Gas herum angeordnet ist,
- eine Ausrüstungshalterungsvorrichtung (1, 11, 12, 13), die imstande und gestaltet ist, um eine medizinische Ausrüstung (3) zu tragen, wobei die Ausrüstungshalterungsvorrichtung (1, 11, 12, 13) umfasst:
• eine Befestigungsstruktur (1, 1a, 1b), die es ermöglicht, die Ausrüstungshalterungsvorrichtung (1, 11, 12, 13) an der Schutzabdeckung (4) aufzuhängen, wobei die Befestigungsstruktur (1, 1a, 1b) einen ersten Befestigungsarm (1a) und einen zweiten Befestigungsarm (1b) umfasst, die einander gegenüber angeordnet sind;
• eine Plattenstruktur (12, 12a, 12b), auf der die zu tragende Ausrüstung (3) angeordnet werden kann,
• ein oder mehrere Anschlusselemente (11, 11a, 11b), die die Befestigungsstruktur (1, 1a, 1b) mit der Plattenstruktur (12, 12a, 12b) verbinden, und
• mindestens eine Haltestruktur (13), die an der Plattenstruktur (12, 12a, 12b) befestigt ist, und ein Halten der Ausrüstung (3) in der Position auf der Plattenstruktur (12, 12a, 12b) ermöglicht,
- und wobei die Ausrüstungshalterungsvorrichtung (1, 11, 12, 13) anhand des ersten (1a) und zweiten Befestigungsarms (1b) an der Schutzabdeckung (4) befestigt ist, **dadurch gekennzeichnet, dass** der erste (1a) und zweite Befestigungsarm (1b) Endabschnitte (111a, 111b) umfassen, die die Abdeckung (4) in der Art einer Rohrschelle einschließen.

2. Anordnung nach dem vorstehenden Anspruch, **dadurch gekennzeichnet, dass** die beiden Befestigungsarme (1a, 1b) an ihren Enden (111a, 111b) in Kontakt mit der Abdeckung (4) mit Einschlussverlängerungen (10) versehen sind, die gestaltet sind, um eine Einschlusskraft auf die Abdeckung (4) auszuüben.

3. Anordnung nach einem der vorstehenden Ansprüche, **dadurch gekennzeichnet, dass** die Endabschnitte (111a, 111b) des ersten (1a) und zweiten Befestigungsarms (1b) oder die Einschlussverlängerungen (10), die den ersten (1a) und zweiten Befestigungsarm (1b) ausrüsten, ausgebildet sind, um sich an die Außenkontur der Abdeckung (4) anzulegen.

4. Anordnung nach einem der vorstehenden Ansprüche, **dadurch gekennzeichnet, dass** die Endabschnitte (111a, 111b) des ersten (1a) und zweiten Befestigungsarms (1b) oder die Einschlussverlängerungen (10) in den Aufnahmen (4a) aufgenommen werden, die in der Schutzabdeckung (4) eingearbeitet sind.

5. Anordnung nach einem der vorstehenden Ansprüche, **dadurch gekennzeichnet, dass** sie ein erstes und ein zweites Anschlusselement (11a, 11b) umfasst, das die Befestigungsstruktur (1, 1a, 1b) mit der Plattenstruktur (12, 12a, 12b) verbindet.

6. Anordnung nach einem der vorstehenden Ansprüche, **dadurch gekennzeichnet, dass** die Plattenstruktur (12, 12a, 12b) einen ersten und einen zweiten Trägerarm (12a, 12b) umfasst.

7. Anordnung nach einem der vorstehenden Ansprüche, **dadurch gekennzeichnet, dass** das erste Anschlusselement (11a) mit dem ersten Befestigungsarm (1a) der Befestigungsstruktur (1, 1a, 1b) und/oder mit dem ersten Trägerarm (12a) der Plattenstruktur (12, 12a, 12b) befestigt oder einteilig damit gebildet ist, und/oder das zweite Anschlusselement (11b) mit dem zweiten Befestigungsarm (1b) der Befestigungsstruktur (1, 1a, 1b) und/oder mit dem zweiten Trägerarm (12b) der Plattenstruktur (12, 12a, 12b) befestigt oder einteilig damit gebildet ist.

8. Anordnung nach einem der vorstehenden Ansprüche, **dadurch gekennzeichnet, dass** der erste und zweite Trägerarm (12a, 12b) durch die gesamte Haltestruktur (13) oder einen Teil davon miteinander verbunden sind.

9. Anordnung nach einem der vorstehenden Ansprüche, **dadurch gekennzeichnet, dass** sie eine medizinische Ausrüstung (3) umfasst, die auf der Plattenstruktur (12, 12a, 12b) angeordnet ist, indem sie dort durch die Haltestruktur (13) gehalten wird.

10. Anordnung nach Anspruch 9, **dadurch gekennzeichnet, dass** die medizinische Ausrüstung (3), die auf der Plattenstruktur (12, 12a, 12b) angeordnet ist, ein Gasbefeuchter ist, der fluidisch an einen Gasausgangsanschluss des Blocks zur Ausgabe (2) von Gas angeschlossen ist, der auf der Gasflasche (5) angeordnet ist.

11. Anordnung nach einem der vorstehenden Ansprüche, **dadurch gekennzeichnet, dass** eine oder mehrere Rückhaltevorrichtungen (15), die aus den Bändern, den Verknüpfungen, den Litzen und den Ketten ausgewählt sind, die Haltestruktur (13) mit dem ersten und zweiten Längsverbindungselement (11a, 11b) verbinden.

12. Verwendung einer Anordnung nach einem der vorstehenden Ansprüche zum Tragen einer medizinischen Ausrüstung (3), wobei die medizinische Ausrüstung (3) auf der Plattenstruktur (12, 12a, 12b) angeordnet ist, indem sie dort durch die Haltestruktur (13) gehalten wird.

13. Verwendung nach Anspruch 12, **dadurch gekennzeichnet, dass** die medizinische Ausrüstung (3) ein Gasbefeuchter ist.

14. Verwendung nach Anspruch 13, **dadurch gekennzeichnet, dass** die medizinische Ausrüstung (3) ein Gasbefeuchter ist, der fluidisch an einen Gasausgangsanschluss (2a) des Blocks zur Ausgabe (2) von Gas angeschlossen ist, der auf der Gasflasche (5) angeordnet ist.

15. Installation zur Bereitstellung von Atemgas für einen Patienten, umfassend:
- eine Anordnung zur Ausgabe von Gas (1, 2, 4, 5) nach einem der Ansprüche 1 bis 11, umfassend eine Gasflasche (5), die mit einem Block zur Ausgabe (2) von Gas ausgerüstet ist, eine Schutzabdeckung (4), die um den Block zur Ausgabe (2) von Gas herum angeordnet ist, und einer Ausrüstungshalterungsvorrichtung (1, 11, 12, 13), auf der ein Gasbefeuchter (3) angeordnet ist, der fluidisch an einen Gasausgangsanschluss (2a) des Blocks zur Ausgabe (2) von Gas angeschlossen ist, ausgerüstet ist, und
- eine flexible Rohrleitung, die fluidisch mit einem Ausgang des Befeuchters (3) verbunden ist,
- und eine Schnittstelle zur Ausgabe von Gas an den Patienten, die fluidisch mit der flexiblen Rohrleitung verbunden ist, wobei die Schnittstelle zur Ausgabe von Gas vorzugsweise eine Atemmaske oder Atembrillen umfasst.

## Claims

1. Gas distribution assembly (1, 2, 4, 5) comprising:
- a gas cylinder (5) equipped with a gas distribution unit (2),
- a protective cap (4) arranged around said gas distribution unit (2),
- an equipment support device (1, 11, 12, 13) capable of and designed to support an item of medical equipment (3), the equipment support device (1, 11, 12, 13) comprises:
• a fixing structure (1, 1a, 1b) making it possible to fasten the equipment support device (1, 11, 12, 13) to the protective cap (4), the fixing structure (1, 1a, 1b) comprising a first fixing arm (1a) and a second fixing arm (1b) arranged facing one another,
• a plate structure (12, 12a, 12b) on which can be arranged said equipment (3) to be supported,
• one or more connection elements (11, 11a, 11b) connecting the fixing structure (1, 1a, 1b) to the plate structure (12, 12a, 12b), and
• at least one holding structure (13) fixed to the plate structure (12, 12a, 12b) and making it possible to hold said equipment (3) in position on said plate structure (12, 12a, 12b),
- and wherein the equipment support device (1, 11, 12, 13) is fixed to said protective cap (4) by way of said first (1a) and second (1b) fixing arms, **characterised in that** said first (1a) and second (1b) fixing arms comprise end portions (111a, 111b) clamping the cap (4) in the manner of a clamping collar.

2. Assembly according to the preceding claim, **characterised in that** the two fixing arms (1a, 1b) are provided at the ends (111a, 111b) thereof in contact with the cap (4) with clamping extenders (10) designed to exert a clamping force on the cap (4).

3. Assembly according to one of the preceding claims, **characterised in that** the end portions (111a, 111b) of the first (1a) and second (1b) fixing arms or clamping extenders (10) equipping said first (1a) and second (1b) fixing arms are shaped to mould the outer edge of the cap (4).

4. Assembly according to one of the preceding claims, **characterised in that** the end portions (111a, 111b) of the first (1a) and second (1b) fixing arms or the extenders (10) are housed in housings (4a) arranged in the protective cap (4).

5. Assembly according to one of the preceding claims, **characterised in that** it comprises a first and a second connection element (11a, 11b) connecting the fixing structure (1, 1a, 1b) to the plate structure (12, 12a, 12b).

6. Assembly according to one of the preceding claims, **characterised in that** the plate structure (12, 12a, 12b) comprises a first and a second supporting arm (12a, 12b).

7. Assembly according to one of the preceding claims, **characterised in that** the first connection element (11a) is fixed to the or formed of one single part with the first fixing arm (1a) of the fixing structure (1, 1a, 1b) and/or with the first supporting arm (12a) of the plate structure (12, 12a, 12b), and/or the second connection element (11b) is fixed to the or formed of one single part with the second fixing arm (1b) of the fixing structure (1, 1a, 1b) and/or with the second supporting arm (12b) of the plate structure (12, 12a, 12b).

8. Assembly according to one of the preceding claims, **characterised in that** said first and second supporting arms (12a, 12b) are connected to one another by all or part of the holding structure (13).

9. Assembly according to one of the preceding claims, **characterised in that** it comprises an item of medical equipment (3) arranged on the plate structure (12, 12a, 12b) by being held there by the holding structure (13).

10. Assembly according to claim 9, **characterised in that** the medical equipment (3) arranged on the plate structure (12, 12a, 12b) is a gas humidifier fluidically connected to a gas outlet connector of the gas distribution unit (2) arranged on the gas cylinder (5).

11. Assembly according to one of the preceding claims, **characterised in that** one or more retaining devices (15), selected from among strips, links, cordons and chains, connect the holding structure (13) to the first and second streamlined connection elements (11a, 11b).

12. Use of an assembly according to one of the preceding claims to support an item of medical equipment (3), said medical equipment (3) being arranged on the plate structure (12, 12a, 12b) by being held there by the holding structure (13).

13. Use according to claim 12, **characterised in that** the medical equipment (3) is a gas humidifier.

14. Use according to claim 13, **characterised in that** the medical equipment (3) is a gas humidifier fluidically connected to a gas outlet connector (2a) of the gas distribution unit (2) arranged on the gas cylinder (5).

15. Installation for supplying breathing gas to a patient comprising:
- a gas distribution assembly (1, 2, 4, 5) according to one of claims 1 to 11 comprising a gas cylinder (5) equipped with a gas distribution unit (2), a protective cap (4) arranged around said gas distribution unit (2), and an equipment support device (1, 11, 12, 13) on which is arranged a gas humidifier (3) fluidically connected to an outlet connector (2a) of the gas distribution unit (2), and
- a flexible pipe fluidically connected to an outlet of the humidifier (3),
- and an interface for distributing gas to the patient fluidically connected to the flexible pipe, preferably the gas distribution interface comprises a breathing mask or breathing glasses.
